# EUROPÄISCHE PATENTSCHRIFT

(11) **EP 1 457 233 B1**
(45) Veröffentlichungstag und Bekanntmachung des Hinweises auf die Patenterteilung: **11.07.2007**
(21) Anmeldenummer: 03005664.2
(22) Anmeldetag: 12.03.2003
(51) Int. Cl.: A61N 1/30

(54) **Transdermales Wirkstoffverabreichungssystem mit Elektrodenraster**
Transdermal drug delivery system with mesh electrode
Système d'administration de médicaments transdermique avec electrode à mailles

(43) Veröffentlichungstag der Anmeldung: 15.09.2004
(73) Patentinhaber: Novosis AG, 83714 Miesbach (DE)
(72) Erfinder: Fischer, Wilfried, Dr., 83714 Miesbach (DE); Haas, Rüdiger, 96181 Leimen (DE); Zimmermann, Clifton, Dr., 76131 Karlsruhe (DE); Meyer, Elisabeth, Dr., 83714 Miesbach (DE)

(56) Entgegenhaltungen:
- EP-A- 0 532 451
- WO-A-00/53256
- WO-A-99/22810
- WO-A-99/52590
- US-A- 5 533 995
- US-A- 5 944 685

## Beschreibung

### 1. Stand der Technik

Auf der permanenten Suche der Pharma-Industrie nach Optimierung der Verabreichung von Arzneimitteln haben transdermale Applikations-Systeme (transdermal delivery systems; TDS) heute einen bedeutenden Platz eingenommen. In bisher realisierten Anwendungsbereichen (z.B. Hormone, Bluthochdruck, Schmerz, Nikotinersatz) haben TDS bereits ein Umsatzvolumen von weltweit über 2 Milliarden US$ erreicht. Bei allen Vorteilen der TDS für Patienten und für das Gesundheitskostensystem ist die Verwendungsmöglichkeit solcher Systeme z.Zt. noch durch Permeabilitätsgrenzen eingeschränkt, die sich aus den chemisch-physikalischen Eigenschaften der zu applizierenden Substanzen ergeben. Viele bekannte Wirkstoffe bieten sich für die transdermale Applikation an, sobald ein System zur Verfügung steht, das z.B. die Permeabilität größerer Moleküle bewirkt. Das zusätzliche Marktpotential ist enorm. Daher gibt es seit einigen Jahren technische Ansätze, die Permeabilität von Substanzen zu verbessern, z.B. die Verwendung von Absorptionsförderern in passiven TDS oder iontophoretische Systeme.

Gewöhnliche TDS nutzen für den Substanz/Wirkstoff-Transport durch die Haut die passive konzentrationsabhängige Diffusion entlang des Konzentrationsgefälles zwischen dem TDS und dem *stratum corneum* der Haut aus. Jedoch gelingt es mit diesem Mechanismus nur, sehr kleine Moleküle durch die Haut zu treiben. Größere, komplexere Moleküle wie Insulin, LH-RH etc. bedürfen einer zusätzlichen treibenden Kraft, um durch die Haut in den Blutkreislauf zu gelangen.

Eine Methode zur Anwendung einer zusätzlichen diffusionserhöhenden Kraft ist die Iontophorese, also der Transport von Molekülen mittels eines angelegten elektrischen Feldes. Hierzu wird zwischen Substanz/Wirkstoff-Träger und Patient eine elektrische Potentialdifferenz erzeugt. Die in Ionenform vorliegenden Moleküle werden dann aus dem leitfähigen Substanz/Wirkstoff-Reservoir durch elektrostatische Abstoßung in die Haut getrieben. Der zeitliche Freisetzungsverlauf der Substanz/des Wirkstoffs läßt sich über entsprechende Ansteuerung der treibenden elektro-motorischen Kraft exakt steuern. Insbesondere bei einem iontophoretischen Insulinsystem ist dies eine kritische Größe. Es ist hierbei aufgrund der geringen therapeutischen Breite des Wirkstoffes absolut notwendig, daß die Freisetzung aus dem System die Permeation durch die Haut kontrolliert.

Zu iontophoretischen Systemen werden externe Steuergeräte geliefert, die über Kabel mit dem System verbunden sind. Es sind ebenfalls aus der Patentliteratur Vorrichtungen bekannt, die aus integrierten Steuereinheiten in Verbindung mit Substanz/Wirkstoff-Reservoir und Elektroden bestehen (s.u.).

Iontophoretische transdermale therapeutische Systeme, wie sie z.B. aus der DE 3703321 C2, WO 92/04938, WO 87/04936, US 3,991,755, US 4,141,359 oder der WO 91/16077 bekannt sind, bestehen im allgemeinen aus einer Kombination zweier Elektroden, wobei eine oder beide Elektroden mit jeweils einem Substanz/Wirkstoff-Reservoir verbunden ist/sind. Mittels einer an beide Elektroden angelegten Spannung können nun, nach Auftragen des iontophoretischen Systems auf die Haut, ionisierte Substanz/Wirkstoffmoleküle durch elektrostatische Abstoßung durch die mit der Substanz/dem Wirkstoff gleichsinnig geladene Elektrode durch die Haut gezwungen werden.

US 5,415,629 beschreibt ein iontophoretisches transdermales Wirkstoff-Applikationssystem mit einer "ionosonic applicator working electrode" bzw. "applicator electrode" 10, die eine Hautberührungsfäche 17 aufweist. Es ist klar zu entnehmen, daß die Elektrode mit der Haut in Berührung steht. Der Wirkstoff wandert also iontophoretisch in einem zwischen Elektrode und Haut liegenden Feld in die Haut. Es werden Maßnahmen angesprochen, um ein Tunneln zu vermeiden, da die Feldliniendichte auf der Haut insbesondere dann unterschiedlich sein kann, wenn die Haut verletzt ist.

EP 0 532 451 betrifft ein transdermales Wirkstoff-Applikationssystem mit einem Elektrodenpaar, bei dem beide Elektroden mit der Schicht in Berührung stehen, die den Wirkstoff enthält. Die beiden Elektroden sind im Abstand voneinander angeordnet, wobei eine wirkstoffhaltige Isolationsschicht den Spalt abdeckt, der von den beiden Elektroden gebildet wird. Der Wirkstoff bewegt sich also in dem elektrischen Feld, das zwischen den beiden Elektroden aufgebaut wird.

Der grundsätzliche Aufbau von iontophoretischen Systemen beinhaltet immer eine Kathode und eine Anode, die dazu dienen, einen direkten Stromfluß durch den Körper zu erzeugen. Demnach müssen die Elektroden in einem derartigen geometrischen Abstand zueinander angebracht sein, daß kein Kurzschluß an der Oberfläche der Haut entstehen kann. Die Elektroden sind dabei mit wässrigen Pufferlösungen, die in Gelen immobilisiert sein können, direkt verbunden. Der elektrische Kontakt zur Haut erfolgt über diese wässrigen Zubereitungen. Ionenhaltige Flüssigkeit daraus kann sich entlang der Oberfläche der Haut ausbreiten und so einen direkten Stromfluß zwischen den Elektroden hervorrufen. Dies bedingt eine gewisse Mindestgröße eines derartigen Systems, um eine Isolation der Elektroden vornehmen zu können.

Zur Vermeidung von Verbrennungen des Hautgewebes, Verhinderung von Polarisation der Elektroden und Hydrolyse des Gewebewassers (ab ca. 1,7 V), die zu einer schmerzhaften pH-Verschiebung führen kann, werden iontophoretische Systeme mit gepulster Gleichspannung oder Wechselspannung betrieben, wobei die Art der Pulse (Form, Höhe, Länge) die Verträglichkeit und Effektivität des iontophoretischen Systems beeinflussen. Das Feld wird großflächig über dem gesamten TDS erzeugt und ist, wenn überhaupt, nur grob regelbar. Damit ist das gesamte System entweder aktiv oder abgeschaltet. Es ist darauf zu achten, daß die Haut zwischen den Spannungs-Applikationen Erholungsphasen benötigt; vgl. z.B. Anesthesiology, 82 (1995) 1146-1153 und J. Pharm. Sci., 87 (1998) 976-981.

Die derzeitigen technischen Ausführungen von iontophoretischen TDS sind sehr aufwendig und teuer. Als Elektroden werden meist edelmetallbeschichtete Metallscheiben verwendet, als Gegenelektroden z.B. Normalelektroden, alles Maßnahmen um evtl. auftretende Polarisationen zu vermeiden. Die Elektrodengele müssen - wie vorstehend ausgeführt - voneinander isoliert angebracht werden und dürfen nicht auslaufen. Alles in allem sind die derzeitigen iontophoretischen TDS groß, teuer und hinsichtlich ihrer Steuerungsmöglichkeiten nicht sehr flexibel. Um jedoch die Vorteile der spannungsgesteuerten Substanz/Wirkstoffpermeation allgemein nutzbar zu machen, bedarf es einfacher, preiswert herzustellender und flexibler TDS.

Auf die in der neueren Zeit beschriebene Elektroporation, die mit sehr kurzen Impulsen (einige ms) und sehr hohen Spannungen (100 -200 V) arbeitet, wird hier nicht näher eingegangen.

Schließlich ist aus US 5 533 995 ein transdermales Applikationssystem mit einer Trägerfolie bzw. Abdeckfolie, einer für zu applizierende Substanzen bzw. Wirkstoffe durchlässige Membran und einem Reservoir mit einem Gehalt an zu applizierenden Substanzen bzw. Wirkstoffen, das zwischen der Trägerfolie und der Membran vorgesehen ist, bekannt, wobei die Trägerfolie eine Elektrode und eine Gegenelektrode umfasst. Die Membran kann auch fehlen. Außerdem kann die Gegenelektrode auch eine der Haut zugewandte Transitchamber tragen. Dieses bekannte Applikationssystem weist zur Befestigung des Systems im Kontakt mit der Haut eines Patienten ein Gehäuse auf, das mit einem Band etwa am Unterarm befestigt wird.

Das System aus US 5 533 995 stellt den Oberbegriff des Anspruchs 1 dar.

### 2. Beschreibung der Erfindung

Aufgabe der vorliegenden Erfindung ist es, ein "intelligentes" elektrisch gesteuertes TDS zur Verfügung zu stellen, das gegenüber dem genannten Stand der Technik verbessert ist.

Die Erfindung betrifft nun ein transdermales Applikationssystem bestehend aus
- einer für zu applizierende Substanzen undurchlässigen Trägerfolie (1), die eine Elektrode oder ein Kollektiv von Elektroden trägt,
- einem Reservoir (5) mit einem Gehalt an zu applizierenden Substanzen, das zwischen der Trägerfolie (1) und einer Gegenelektrode bzw. einem Kollektiv von Gegenelektroden vorgesehen ist,
- einer für die zu applizierbaren Substanzen durchlässigen Membran (10), die die Gegenelektrode bzw. das Kollektiv von Gegenelektroden umfasst, wobei das Applikationssystem eine Batterie, einen integrierten Controller und ein Lese- und Schreibgerät zum Beschreiben des integrierten Controllers aufweist dadurch gekennzeichnet, daß eine Abzienfolie vorhanden ist und dadurch, daß auf der dem Substanz-Reservoir abgewandten Seite der Gegenelektrode bzw. den Kollektiv von Gegenelektroden oder der dem Substanz-Reservoir abgewandten Seite der Membran eine Schicht aus einem druckempfindlichen Haftkleber zur Befestigung des Applikationssystems auf der Haut und zur Verbesserung des Hautkontakts vorgesehen ist, und wobei der controller auf der Trägerfolie fixiert ist.

Zur Anwendung dieses Applikationssystems wird die Membran in Kontakt mit der Haut gebracht. Dann wird in einem ersten Schritt zwischen Elektrode und Membran, d.h. im Bereich des Substanz-Reservoirs, ein elektrisches Feld angelegt. Daraufhin entsteht zu beiden Seiten der Membran eine Substanz schicht mit einer Substanz konzentration, wie sie aufgrund von Sättigungseffekten und der damit verbundenen beschränkten Lagerfähigkeit für ein direkt hergestelltes Substanz-Reservoir nicht möglich ist. Nach Abschalten des Feldes wird die Substanz aufgrund des Konzentrationsgefälles von der Haut absorbiert.

Eine weitere Ausführungsform des erfindungsgemäßen Applikationssystems ist dadurch gekennzeichnet, daß die Elektrode bzw. Elektroden der Trägerfolie und/oder der Membran aufgedruckt ist bzw. sind.

Eine weitere Ausführungsform des erfindungsgemäßen Applikationssystems ist dadurch gekennzeichnet, daß die elektrisch leitfähige, für eine Substanz durchlässige Membran eine Elektrode bzw. Elektroden umfaßt, indem die Membran als ein Metallgitter oder ein mit einer leitfähigen Schicht bedampftes Polymergitter oder eine gelochte leitfähige Folie ausgebildet ist.

Eine weitere Ausführungsform des erfindungsgemäßen Applikationssystems ist dadurch gekennzeichnet, daß die elektrisch leitfähige, für eine Substanz durchlässige Membran eine Elektrode bzw. Elektroden umfaßt, indem die Membran durchgehend leitfähig ist oder leitfähige Bereiche aufweist, die durch nicht leitfähige Bereiche getrennt sind, so daß ein oder mehrere Raster aus leitfähigen Bereichen gebildet werden.

Eine Ausführungsform des erfindungsgemäßen Applikationssystems ist dadurch gekennzeichnet, daß die Elektroden der Trägerfolie in Form von einem oder mehreren Elektrodenraster(n) auf der Trägerfolie angeordnet sind, wobei jedes Elektrodenraster eine Vielzahl von Einzelelektroden aufweist, und die Membran die Gegenelektroden aufweist.

Eine weitere Ausführungsform des erfindungsgemäßen Applikationssystems ist gekennzeichnet durch eine Trägerfolie einer Dicke im Bereich von 10 bis 1000 µm.

Eine weitere Ausführungsform des erfindungsgemäßen Applikationssystems ist dadurch gekennzeichnet, daß das Substanz-Reservoir durch einen Haftkleber, ein Gel oder eine immobilisierte Lösung für die Substanz gebildet wird.

Der Microchip bzw. Microcomputer kann personifizierbar sein; er kann einen Barcode tragen, der Patientendaten kodiert und mit Hilfe eines Lesegerätes ausgelesen werden kann.

Eine weitere Ausführungsform des erfindungsgemäßen Applikationssystems ist dadurch gekennzeichnet, daß im Falle von mehreren Elektrodenrastern jedes Elektrodenraster für sich ansteuerbar ist oder mehrere Elektrodenraster in Gruppen ansteuerbar sind und/oder jede Einzelelektrode in einem Raster für sich ansteuerbar ist oder mehrere Einzelelektroden in einem Raster in Gruppen ansteuerbar sind.

Eine weitere Ausführungsform des erfindungsgemäßen Applikationssystems ist dadurch gekennzeichnet, daß jeder Elektrode der Trägerfolie eine jeweils nur ihr zugeordnete Gegenelektrode entspricht.

Eine weitere Ausführungsform des, erfindungsgemäßen Applikationssystems ist dadurch gekennzeichnet, daß der Microcomputer, insbesondere der Integrierte Controller, mit der Trägerfolie fest verschweißt ist.

Eine weitere Ausführungsform des erfindungsgemäßen Applikationssystems ist dadurch gekennzeichnet, daß es sich bei dem Microcomputer, insbesondere dem Integrierten Controller, um einen rezeptbezogen programmierbaren Chip handelt.

Eine weitere Ausführungsform des erfindungsgemäßen Applikationssystems ist dadurch gekennzeichnet, daß es sich bei der Batterie um eine Knopf- oder Folienbatterie handelt.

Eine weitere Ausführungsform des erfindungsgemäßen Applikationssystems ist dadurch gekennzeichnet, daß die Batterie in einer Tasche der Trägerfolie vorgesehen ist. Vorzugsweise ist die Batterie wieder aufladbar.

Bei dem erfindungsgemäßen Applikationssystem können die Elektrode(n) und/oder Gegenelektrode(n) als Gitter ausgebildet sein.

Ferner können bei dem erfindungsgemäßen Applikationssystem die Elektrode(n) und/oder Gegenelektrode(n) aus metallischem, graphitischem oder elektrisch leitendem polymeren Material bestehen.

Ferner kann das erfindungsgemäße Applikationssystem für einen Stromfluß von 0,01 bis 10 mA/cm² ausgelegt sein.

Ferner kann bei dem erfindungsgemäßen Applikationssystem das Reservoir als austauschbares Element ausgebildet sein.

Ferner kann bei dem erfindungsgemäßen Applikationssystem die Trägerfolie mit Elektrode(n) und/oder die Membran mit Gegenelektrode (n) als austauschbares Element bzw. austauschbare Elemente ausgebildet sein.

Ferner kann bei dem erfindungsgemäßen Applikationssystem die Trägerfolie mit Elektrode(n) und/oder die Gegenelektrode bzw. das Kollektiv von Gegenelektroden als austauschbares Element bzw. austauschbare Elemente ausgebildet sein.

Schließlich kann bei dem erfindungsgemäßen Applikationssystem der Integrierte Controller als austauschbares Element ausgebildet sein.

Im folgenden werden die einzelnen Bestandteile des erfindungsgemäßen transdermalen Applikationssystems ohne Beschränkung detaillierter beschrieben.

### 2.1 Die Trägerfolie

Die Größe des erfindungsgemäßen TDS muß sich nicht von der Größe herkömmlicher passiver TDS (einige 10 cm²) unterscheiden. Die Trägerfolie erhält zur Herstellung einer Ausführungsform der Erfindung durch z.B. Bedruckung (Ätzung) ein Raster von flächigen oder flächenförmigen Elektroden, deren Geometrie so ausgestaltet ist, daß mit den Gegenelektroden der Membran ein konzentriertes elektrisches Feld im Bereich der jeweiligen Antipoden entsteht. Die Feldgeometrie sowie die Höhe der angelegten Potential-Differenz ist so ausgelegt, daß die Substanz/Wirkstoff-Ionen aus der Substanz/Wirkstoffmatrix in die Haut getrieben werden. Die Ansteuerung der einzelnen Elektroden erfolgt rasterförmig, systematisch bzw. über einen Zufallsgenerator.

### 2.2. Integrierter Controller

Am Rand der Trägerfolie kann sich ein aufgeschweißter Integrierter Controller ("Controller") mit (optional) elektromagnetisch wiederbeschreibbarem Speicher befinden. Dieser Controller kann die o.g. Elektroden rezeptbezogen ansteuern, wobei die (optional) individuellen Patienten-Parameter im Therapieverlauf berücksichtigt werden können. Hierzu kann der Patient oder der behandelnde Arzt ein Kartenlese- und Kartenschreibgerät erhalten. Vor jeder Behandlung können die individuellen Patienten-Daten über das Lesegerät in den Controller-Speicher übertragen werden. Abhängig von den eingegebenen Daten kann der Patient nun über das so programmierte TDS die Wirkstoffe in einer optimalen Dosierung erhalten.

### 2.3 Energiequelle

Als Energiequelle können eine oder mehrere Knopfzellen oder entsprechende Folienbatterien verwendet werden. Hierfür kann die Trägerfolie z.B. in unmittelbarer Nähe des Integrierten Controllers eine Tasche mit entsprechenden Anschlüssen aufweisen, in die die Batterie eingeführt werden kann. Die Leistung einer solchen Zelle wird, je nach Substanz/Wirkstoff und Anwendungsdauer, für eine oder mehrere TDS-Applikationen ausreichen, so daß die Batterien in der Regel mehrfach verwendet werden können.

### 2.4 Schreib-/Lesegerät

Dessen Funktion wurde bereits oben unter 2.2 beschrieben.

### 2.5 Unterschiede zu iontophoretischen Systemen

Das erfindungsgemäße TDS kann nach einer Ausführungsform z.B. aus einer Vielzahl von Elektrodenpaaren bestehen, die auf die Ober- oder Unterseite der Trägerfolie und/oder der Membran des TDS aufgedruckt sind (Elektrodenraster). Durch diese Elektrodenausbildung ist es möglich, große Stückzahlen an Elektroden bzw. TDS sehr kostengünstig mittels Standard-Druckverfahren herzustellen. Die bedruckte Folie büßt ihre Flexibilität nicht ein. Der Abstand der Gegenelektroden sollte bei dieser Ausführungsform sehr präzise bestimmt werden. Der Abstand der Elektrodenpaare in lateraler Richtung kann sehr einfach durch das Druckmuster variiert werden. Der Abstand der Elektrodenpaare voneinander kann so ausgelegt sein, daß sich eine getrennte Ansteuerung der Einzelpaare realisieren läßt. Die Elektrodenfläche beträgt typischerweise 0,1 bis 50 cm².

Die Art der Applikation des elektrischen Felds in dem erfindungsgemäßen TDS erlaubt erstmals eine variable Zustandsänderung beliebiger Flächen des TDS. So können Teile des TDS positiv geladen sein, während gleichzeitig andere negativ oder ungeladen sind. Es kann über das TDS ein wiederkehrendes Muster der Verteilung des elektrischen Feldes aufgebaut werden, welches entlang der Elektrodenraster bzw. der Elektroden oder Elektrodenpaare linienförmig oder flächig ausgebildet sein kann. Damit ist es möglich, sehr individuell auf die Feldstärken-Anforderungen unterschiedlicher Substanzen/Wirkstoffe einzugehen, z.B. Teilbereiche des TDS zu aktivieren, während sich andere Bereiche im Ruhezustand befinden, so daß sich die Haut dort "erholen" kann.

Ein grundsätzlicher Unterschied zu iontophoretischen Systemen besteht darin, daß ionisierte Moleküle entlang der Feldlinien innerhalb des Substanz/Wirkstoff-Reservoirs wandern. Dies kann in Medien mit geringer elektrischer Leitfähigkeit oder in elektrisch nicht leitfähigen Medien erfolgen. Damit ist es möglich, gegensinnig geladene Elektroden ohne speziellen Isolator innerhalb einer Ebene des TDS anzuordnen. Wenn die Feldstärke des elektrischen Feldes so groß gewählt wird, daß sich im Bereich der Membran eine erhöhte Konzentration an Substanz/Wirkstoffmolekülen aufbaut, wird die Penetration von geladenen Substanz/Wirkstoffmolekülen in die Haut beeinflußt, wobei im allgemeinen eine Penetrationsverstärkung erwünscht ist.

Die Elektrodenraster, Elektroden oder Elektrodenpaare können dabei mit konstanter oder gepulster Gleichspannung oder Wechselspannung unterschiedlicher Wellenformen betrieben werden.

Da die Haut nicht mit den Elektroden in Kontakt kommt, und kein Stromfluß durch die Haut stattfinde, kann es unter Anwendung des Elektrodenraster-TDS nicht zu Verbrennungen oder zur Hydrolyse von Gewebswasser kommen. Die Hautverträglichkeit der erfindungsgemäßen Systeme ist dementsprechend gegenüber iontophoretischen Systemen deutlich verbessert.

Als Trägerfolien können Folien aus beispielsweise Polyester, Polyethylen oder Polypropylen mit Stärken von 10 bis 1000 µm verwendet werden.

Die Elektroden können aus Kupfer, Silber, Gold, Platin oder anderen leitfähigen Materialien sein, die auf die Trägerfolie und Membran mittels entsprechender Druckverfahren wie Tiefdruck, Siebdruck oder Ätzung aufgebracht werden können.

Das Substanz/Wirkstoff-Reservoir kann ein substanz/wirkstoffhaltiger druckempfindlicher Haftkleber, ein substanz/wirkstoffhaltiges Gel oder eine immobilisierte Substanz/Wirkstofflösung sein, wobei deren pH-Wert die Ionisierung der betreffenden Substanz/des betreffenden Wirkstoffes ermöglichen kann.

Der Fachmann kann sich beispielsweise an folgenden Stand der Technik halten: DE 3 703 321 (Flüssigreservoir und Matrix), DE 4 040 911 (Gel und ionische Lösung), EP 0 532 451 (Gel und Hydrogel), WO 93/03 790 (wässerige Lösung und Polymermatrix) WO 94/16765 (Gel), US 5 160 316 (leitfähige Matrix) und US 5 415 629 (leitfähige Matrix).

Als Substanzen/Wirkstoffe können Substanzen/Wirkstoffe aus der Klasse der Opioide, Antiasthmatika, regulatorischen Peptide, Parasympathomimetika, Parasympatholytika oder Lokalanaesthetika, ohne hierauf beschränkt zu sein, eingesetzt werden. Die Konzentrationen der Substanzen/Wirkstoffe in den Reservoirs kann in weiten Grenzen variiert werden, und sie hängen in unterschiedlichem Ausmaß von der gewünschten Freisetzungsrate und der notwendigen Permeation durch Haut ab.

Typische Konzentrationen liegen im Bereich von 0,01 bis 10 % der Gesamtmasse des Reservoirs. Die Hautpermeation der betreffenden Substanzen/Wirkstoffe kann durch Zumischung üblicher Permeationsförderer noch beeinflußt werden.

Von den Figuren zeigen
**Figur 1:** eine schematische Darstellung eines Elektrodenraster-TDS mit Integriertem Controller,

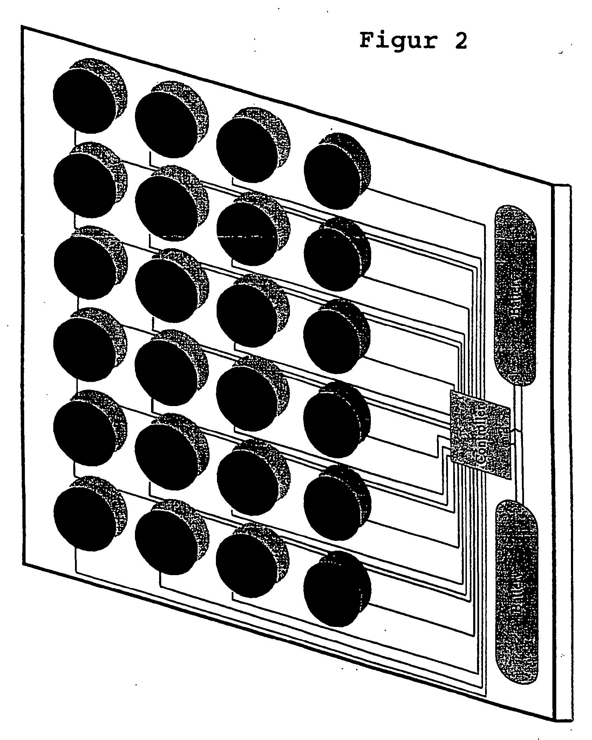

**Figur 2:** eine schematische Darstellung eines Elektrodenraster-TDS mit integriertem Controller und Batterien,
**Figur 3:** eine schematische Darstellung der Wirkungsweise einer Ausführungsform der Erfindung mit einem Substanz/Wirkstoff-Reservoir zwischen einer Trägerfolie mit Elektrodenraster (nicht näher dargestellt) und einer für die Substanz/den Wirkstoff durchlässigen Membran gleichfalls mit Elektrodenraster,
**Figur 4** eine schematische Darstellung der Wirkungsweise einer weiteren Ausführungsform der Erfindung mit einem Substanz/Wirkstoff-Reservoir zwischen einer Einzelelektrode und einer als Gegenelektrode fungierenden und für die Substanz/den Wirkstoff durchlässigen Membran, und

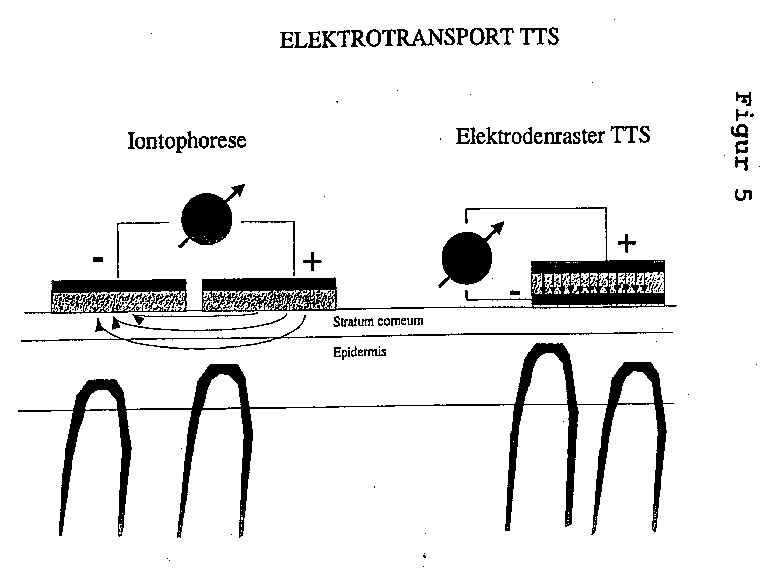

**Figur 5:** einen Vergleich der Wirkungsweise eines iontophoretischen Systems mit dem erfindungsgemäßen Elektrodenraster-TDS.

Die folgenden Beispiele erläutern die Erfindung ohne Beschränkung detaillierter.

### Beispiele

### Beispiel 1

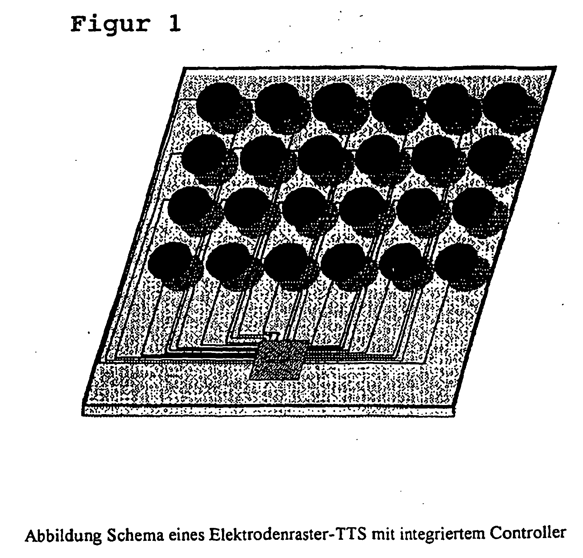

Auf eine Polyesterfolie einer Dicke von 100 µm und auf einer durchlässigen Membran werden jeweils einseitig kreisflächenförmige Kupfer-Elektroden mit einem Durchmesser von 6 mm aufgedruckt (analog **Figur 1**) und anschließend galvanisch versilbert. Jede Elektrode ist mit einer auf die Folie bzw. die Membran aufgedruckten Zuleitung, die ebenfalls versilbert ist, versehen. Die voneinander isolierten Zuleitungen werden jeweils individuell mit einem Steuergerät (Controller) elektrisch leitend verbunden. Der Controller erzeugt pulsierende Gleichspannungen im Bereich bis 7,5 V, wobei die Frequenz der Spannung von 0 bis 2 kHz variiert werden kann. Als Pulsform kommen Sinushalbwellen, Dreieck- oder Rechteckimpulse in Frage. Der Controller kann dabei ebenfalls auf die Trägerfolie aufgedruckt werden oder zusammen mit der benötigten Energiequelle in einem externen Gehäuse untergebracht sein. In letzterem Falle werden die Elektroden über eine flexible Verdrahtung mit dem Controller verbunden. Jedes Elektrodenpaar kann individuell angesteuert werden.

In der Lösung eines druckempfindlichen Haftklebers (Duro-Tak 287-2097, unvernetzter Acrylatkleber ohne funktionelle Gruppen), wird ein ionischer Farbstoff (als Modellstoff für einen ionischen Arzneistoff) gelöst, so daß seine Konzentration in der Trockenmasse 0,25 % beträgt.

Auf eine Seite der elektrodentragenden Folie wird mit dem Fachmann bekannten Beschichtungsverfahren (z.B. Rakelverfahren) eine druckempfindliche Haftschicht aus der oben beschriebenen Farbstoff/Klebstofflösung heraus aufgebracht und das Lösungsmittel mittels Warmluft verdunstet. Danach wird die durchlässige Membran auf die Klebstoffschicht aufgebracht. Gegebenenfalls wird danach analog eine weitere Klebstoffschicht auf die der Haut zuzuwendende Seite der Membran aufgebracht.

Durch Anlegen einer einheitlichen Spannung von z.B. 6 V und 0 Hz an alle Elektroden wird die Diffusion des Farbstoffes in der Klebstoffschicht beschleunigt.

### Beispiel 2

Es wird analog Beispiel 1 eine Polyesterfolie mit beispielsweise einem oberen, der klebstoffhaltigen Seite abgewandten Elektrodenraster versehen. Eine durchlässige Membran wird mit ringförmigen Elektroden bedruckt, wobei die Ringe den flächenförmigen Elektroden genau gegenüber liegen. Die ringförmigen Elektroden werden gemeinsam verbunden und sind damit nicht individuell ansteuerbar. Diese Elektroden stellen das Nullpotential dar. Aus der Folie und der Membran werden kreisrunde, 8 Elektroden tragende Stücke mit einer Fläche von 5 cm² herausgestanzt.

500 mg Agarose werden zusammen mit 200 mg Hydromorphon-Hydrochlorid in 9,3 g Wasser bei 90 °C gelöst. Man kühlt die Lösung auf 65 °C ab und streicht sie mit einem vorgewärmten Auftragsrakel zu einer 0,4 mm starken Schicht aus und läßt sie erkalten. Nach dem Erkalten werden kreisförmige 5 cm² große Stücke aus der Gelschicht ausgestanzt und auf der Unterseite der oben beschriebenen Elektrodenfeld-Folien und der Oberseite der herausgestanzten Membranen mittels eines Klemmringes so befestigt, daß zwischen Gelschicht und jeweiliger Folie und Membran keine Lufteinschlüsse auftreten können.

Durch Variation der Spannung (2 bis 200 V) kann der Substanz/Wirkstoffstrom im Hydrogel bzw. die Stärke der Substanz/Wirkstoffbewegung durch temporäres Aktivieren/Deaktivieren einzelner Elektrodenpaare moduliert werden.

### Beispiel 3

Die gestanzte Elektrodenrasterfolie aus Beispiel 2 wird mit einer überstehenden Folie (Hostaphan MN 19), die einseitig mit einem selbstklebenden Haftkleber (Duro-Tak 287-2287) beschichtet ist, derart überklebt, daß sich ein gleichmäßiger Klebering ergibt. Nach der Montage des substanz/wirkstoffhaltigen Gels und der gestanzten Membran wird das Elektrodenraster-TDS auf eine silikonisierte Schutzfolie aufgebracht. Die Schutzfolie wird vor dem Aufkleben des Systems auf die Haut entfernt.

### Beispiel 4

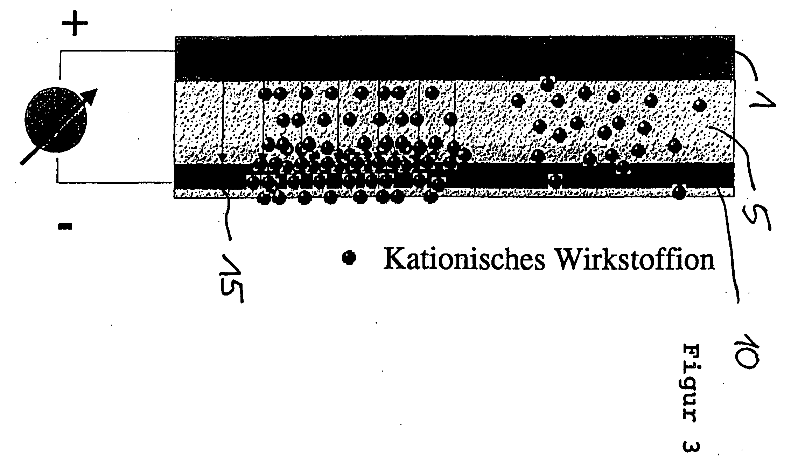

Eine weitere Ausführungsform des erfindungsgemäßen Applikationssystems ist in **Figur 3** dargestellt. Das Bezugszeichen 1 bezeichnet einen Elektrodenträger (Trägerfolie, zugleich Abdeckfolie) mit nicht näher dargestelltem Elektrodenraster. Das Bezugszeichen 5 bezeichnet das Substanz/Wirkstoffreservoir. Das Bezugszeichen 10 bezeichnet die durchlässige Membran mit Gegenelektroden-Raster und das Bezugszeichen 15 den Haftkleber. Die Abziehfolie ist nicht dargestellt.

### Beispiel 5

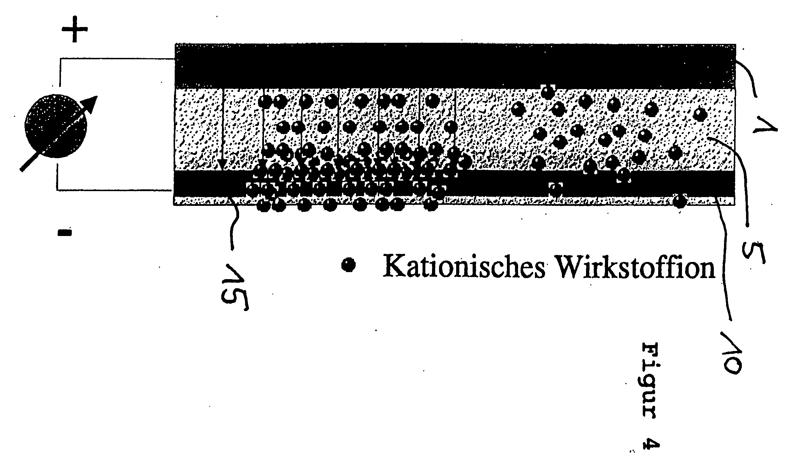

Eine weitere Ausführungsform des erfindungsgemäßen Applikationssystems ist in **Figur 4** dargestellt. Das Bezugszeichen 1 bezeichnet eine Einzelelektrode. Das Bezugszeichen 5 bezeichnet das Substanz/Wirkstoffreservoir. Das Bezugszeichen 10 bezeichnet die durchlässige Membran mit Gegenelektrode und das Bezugszeichen 15 den Haftkleber. Die Abziehfolie ist nicht dargestellt.

## Patentansprüche

1. Transdermales Applikationssystem bestehend aus
- einer für zu applizierende Substanzen undurchlässigen Trägerfolie (1), die eine Elektrode oder ein Kollektiv von Elektroden trägt,
- einem Reservoir (5) mit einem Gehalt an zu applizierenden Substanzen das zwischen der Trägerfolie (1) und einer Gegenelektrode bzw. einem Kollektiv von Gegenelektroden vorgesehen ist,
- einer für die zu applizierbaren Substanzen durchlässigen Membran (10), die die Gegenelektrode bzw. das Kollektiv von Gegenelektroden umfasst, wobei das Applikationssystem eine Batterie, einen integrierten Controller und ein Lese- und Schreibgerät zum Beschreiben des integrierten Controllers aufweist **dadurch gekennzeichnet, daß**
eine Abziehfolie vorgesehen ist und dadurch, daß auf der dem Substanz -Reservoir abgewandten Seite der Gegenelektrode bzw. dem Kollektiv von Gegenelektroden oder der dem Substanz-Reservoir abgewandten Seite der Membran eine Schicht aus einem druckempfindlichen Haftkleber zur Befestigung des Applikationssystems auf der Haut und zur Verbesserung des Hautkontakts vorgesehen ist, und wobei der Controller auf der Trägerfolie fixiert ist.

2. Applikationssystem nach Anspruch 1, **dadurch gekennzeichnet, daß** die Elektrode bzw. Elektroden der Trägerfolie und/oder der Membran aufgedruckt ist bzw. sind.

3. Applikationssystem nach einem der vorhergehenden Ansprüche, **dadurch gekennzeichnet, daß** die für eine Substanz durchlässige Membran eine Elektrode bzw. Elektroden umfaßt, indem die Membran als ein Metallgitter oder ein mit einer leitfähigen Schicht bedampftes Polymergitter oder eine gelochte leitfähige Folie ausgebildet ist.

4. Applikationssystem nach einem der vorhergehenden Ansprüche, **dadurch gekennzeichnet, daß** die für eine Substanz durchlässige Membran eine Elektrode bzw. Elektroden umfaßt, indem die Membran durchgehend leitfähig ist oder leitfähige Bereiche aufweist, die durch nicht leitfähige Bereiche getrennt sind, so daß ein oder mehrere Raster aus leitfähigen Bereichen gebildet werden.

5. Applikationssystem nach einem der vorhergehenden Ansprüche, **dadurch gekennzeichnet, daß** die Elektroden der Trägerfolie als Kollektiv in Form von einem oder mehreren Elektrodenraster(n) auf der Trägerfolie angeordnet sind, wobei jedes Elektrodenraster eine Vielzahl von Einzelelektroden aufweist, und auf der gegenüberliegenden Seite des Reservoirs die Gegenelektroden vorgesehen sind.

6. Applikationssystem nach einem der vorhergehenden Ansprüche, **gekennzeichnet durch** eine Trägerfolie einer Dicke im Bereich von 10 bis 1000 µm.

7. Applikationssystem nach einem der vorhergehenden Ansprüche, **dadurch gekennzeichnet, daß** das Substanz-Reservoir durch einen Haftkleber, ein Gel oder eine immobilisierte Lösung für die Substanz gebildet wird.

8. Applikationssystem nach einem der vorhergehenden Ansprüche, **dadurch gekennzeichnet, daß** im Falle von mehreren Elektrodenrastern jedes Elektrodenraster für sich ansteuerbar ist oder mehrere Elektrodenraster in Gruppen ansteuerbar sind und/oder jede Einzelelektrode in einem Raster für sich ansteuerbar ist oder mehrere Einzelelektroden in einem Raster in Gruppen ansteuerbar sind.

9. Applikationssystem nach einem der vorhergehenden Ansprüche und insbesondere nach Anspruch 4, **dadurch gekennzeichnet, daß** jeder Elektrode der Trägerfolie eine jeweils nur ihr zugeordnete Gegenelektrode entspricht.

10. Applikationssystem nach einem der vorhergehenden Ansprüche, **dadurch gekennzeichnet, daß** der Integrierte Controller mit der Trägerfolie fest verschweißt ist.

11. Applikationssystem nach einem der vorhergehenden Ansprüche, **dadurch gekennzeichnet, daß** es sich bei dem Integrierten Controller um einen rezeptbezogen programmierbaren Chip handelt.

12. Applikationssystem nach einem der vorhergehenden Ansprüche, **dadurch gekennzeichnet, daß** es sich bei der Batterie um eine Knopf- oder Folienbatterie handelt.

13. Applikationssystem nach einem der vorhergehenden Ansprüche, **dadurch gekennzeichnet, daß** die Batterie in einer Tasche der Trägerfolie vorgesehen ist.

14. Applikationssystem nach einem der vorhergehenden Ansprüche, bei dem die Elektrode(n) und/oder Gegenelektrode(n) als Gitter ausgebildet sind.

15. Applikationssystem nach einem der vorhergehenden Ansprüche, bei dem die Elektrode(n) und/oder Gegenelektrode(n) aus metallischem, graphitischem oder elektrisch leitendem polymeren Material bestehen.

16. Applikationssystem nach einem der vorhergehenden Ansprüche, wobei das System für einen Stromfluß von 0,01 bis 10 mA/cm² ausgelegt ist.

17. Applikationssystem nach einem der vorhergehenden Ansprüche, wobei das Reservoir als austauschbares Element ausgebildet ist.

18. Applikationssystem nach mindestens einem der vorhergehenden Ansprüche, wobei die Trägerfolie mit Elektrode(n) und/oder die Membran mit Gegenelektrode(n) als austauschbares Element bzw. austauschbare Elemente ausgebildet sind.

19. Applikationssystem nach mindestens einem der Ansprüche 1 bis 16, wobei die Trägerfolie mit Elektrode(n) und/oder die Gegenelektrode bzw. das Kollektiv von Gegenelektroden als austauschbares Element bzw. austauschbare Elemente ausgebildet sind.

20. Applikationssystem nach mindestens einem der vorhergehenden Ansprüche, wobei der Integrierte Controller als austauschbares Element ausgebildet ist.

## Claims

1. Transdermal delivery system consisting of
- a carrier layer (1) impermeable to the substances to be delivered, which carrier layer carries one electrode or a collective of electrodes;
- a reservoir (5) containing substances to be delivered, the reservoir being provided between the carrier layer (1) and a counter electrode or a collective of counter electrodes;
- a membrane (10) permeable to the substances to be delivered, which membrane comprises the counter electrode or the collective of counter electrodes, wherein the delivery system comprises a battery, a microchip, and a reading and writing device for writing to the microchip, **characterised in that** a removable film is provided, and **in that** there is provided on the side of the counter electrode or the collective of counter electrodes remote from the substance reservoir or on the side of the membrane remote from the substance reservoir, a layer consisting of a pressure-sensitive contact adhesive for attaching the delivery system to the skin and for improving contact with the skin, and wherein the microchip is fixed to the carrier layer.

2. Delivery system according to claim 1, **characterised in that** the electrode(s) of the carrier layer and/or the membrane has/have been applied by printing.

3. Delivery system according to any one of the preceding claims, **characterised in that** the membrane permeable to a substance to be delivered comprises (an) electrode(s), wherein the membrane is a metal lattice, or a polymer lattice on which a conductive layer has been vapour-deposited, or a perforated conductive layer.

4. Delivery system according to any one of the preceding claims, **characterised in that** the membrane permeable to a substance to be delivered comprises (an) electrode(s), wherein the membrane has uninterrupted conductivity or has conductive areas that are separated by non-conductive areas, so that one or more networks of conductive areas are formed.

5. Delivery system according to any one of the preceding claims, **characterised in that** the electrodes of the carrier layer are arranged in the form of one ore more electrode network(s) on the carrier layer, each electrode network having a plurality of individual electrodes, and the counter electrode is provided on the side opposite to the reservoir.

6. Delivery system according to any one of the preceding claims, **characterised by** a carrier layer having a thickness in the range from 10 to 1000 µm.

7. Delivery system according to any one of the preceding claims, **characterised in that** the substance reservoir is formed by a contact adhesive, a gel or an immobilised solution for the substance.

8. Delivery system according to any one of the preceding claims, **characterised in that**, where there is a plurality of electrode networks, each electrode network is actuatable individually or a plurality of electrode networks are actuatable in groups and/or each individual electrode in a network is actuatable individually or a plurality of individual electrodes in a network are actuatable in groups.

9. Delivery system according to any one of the preceding claims and, especially, according to claim 4, **characterised in that** each electrode of the carrier layer corresponds to a counter electrode with which it is uniquely associated.

10. Delivery system according to any one of the preceding claims, **characterised in that** the microchip is securely bonded to the carrier layer.

11. Delivery system according to any one of the preceding claims, **characterised in that** the microchip is a chip that is programmable according to a prescription.

12. Delivery system according to any one of the preceding claims, **characterised in that** the battery is a button battery or sheet battery.

13. Delivery system according to any one of the preceding claims, **characterised in that** the battery is provided in a pocket in the carrier layer.

14. Delivery system according to any one of the preceding claims, where the electrode(s) and/or counter electrode(s) is/are a lattice.

15. Delivery system according to any one of the preceding claims, where the electrode(s) and/or counter electrode(s) consist(s) of a metal material, graphite material or electrically conductive polymer material.

16. Delivery system according to any one of the preceding claims, wherein the system is designed for a current flow of from 0,01 to 10 mA/cm².

17. Delivery system according to any one of the preceding claims, wherein the reservoir is designed as interchangeable element.

18. Delivery system according to at least one of the preceding claims, wherein the carrier layer having electrode(s) and/or the membrane having counter electrode(s) is/are designed as interchangeable element(s).

19. Delivery system according to at least one of claims 1 to 16, wherein the carrier layer having (an) electrode(s) and/or the counter electrode or the collective of counter electrodes is/are designed as interchangeable element(s).

20. Delivery system according to at least one of the preceding claims, wherein the microchip is designed as interchangeable element.

## Revendications

1. Système d'administration transdermique se composant de :
- une plaque support (1) non perméable aux substances à appliquer qui porte une électrode ou un ensemble d'électrodes,
- un réservoir (5) avec une teneur en substances à appliquer qui est prévu entre la plaque support (1) et une contre-électrode et/ou un ensemble de contre-électrodes,
- une membrane (10) perméable aux substances à appliquer qui comprend les contre-électrodes et/ou l'ensemble de contre-électrodes, le système d'administration comprenant une batterie, un contrôleur intégré et un appareil de lecture et d'enregistrement pour écrire sur le contrôleur intégré, **caractérisé en ce qu'**une pellicule détachable est prévue et **en ce qu'**une couche d'adhésif de contact sensible à la pression servant à fixer le système d'administration sur la peau et à améliorer le contact avec la peau est prévue sur le côté de la contre-électrode opposé au réservoir à substance ou sur le côté de la membrane opposé au réservoir à substance, et auquel cas le contrôleur est fixé sur la plaque support.

2. Système d'administration selon la revendication 1, **caractérisé en ce que** l'électrode et/ou les électrodes de la plaque support et/ou de la membrane est ou sont imprimée(s).

3. Système d'administration selon l'une des revendications précédentes, **caractérisé en ce que** la membrane perméable à une substance comprend une électrode et/ou des électrodes, tandis que la membrane est conçue comme une maille métallique ou une maille en polymères vaporisée d'une couche conductrice ou une plaque conductrice perforée.

4. Système d'administration selon l'une des revendications précédentes, **caractérisé en ce que** la membrane perméable à une substance comprend une électrode et/ou des électrodes, tandis que la membrane est totalement conductrice ou comprend des secteurs conducteurs qui sont séparés par des secteurs non conducteurs, de sorte qu'une ou plusieurs matrices sont formées de secteurs conducteurs.

5. Système d'administration selon l'une des revendications précédentes, **caractérisé en ce que** les électrodes de la plaque support sont disposées comme un ensemble sous forme d'une ou plusieurs matrice(s) d'électrode(s) sur la plaque support, chaque matrice d'électrodes comprenant une multitude d'électrodes isolées, et les contre-électrodes sont prévues sur le côté opposé du réservoir.

6. Système d'administration selon l'une des revendications précédentes, **caractérisé par** une plaque support avec une épaisseur comprise dans un secteur allant de 10 à 1000 µm.

7. Système d'administration selon l'une des revendications précédentes, **caractérisé en ce que** le réservoir à substance est formé d'un adhésif de contact, d'un gel ou d'une solution immobilisée pour la substance.

8. Système d'administration selon l'une des revendications précédentes, **caractérisé en ce que** dans le cas de plusieurs matrices d'électrodes chaque matrice d'électrodes peut être amorcée individuellement, ou plusieurs matrices d'électrode peuvent être amorcées en groupes et/ou chaque électrode isolée dans une matrice peut être amorcée individuellement ou plusieurs électrodes isolées dans une matrice peuvent être amorcées en groupes.

9. Système d'administration selon l'une des revendications précédentes et en particulier selon la revendication 4, **caractérisé en ce qu'**à chaque électrode de la plaque support correspond respectivement une contre-électrode qui n'est affectée qu'à elle.

10. Système d'administration selon l'une des revendications précédentes, **caractérisé en ce que** le contrôleur intégré est solidement soudé avec la plaque support.

11. Système d'administration selon l'une des revendications précédentes, **caractérisé en ce qu'**il s'agit, pour le contrôleur intégré, d'une puce programmable liée à la prescription.

12. Système d'administration selon l'une des revendications précédentes, **caractérisé en ce qu'**il s'agit, pour la batterie, d'une batterie à bouton ou d'une batterie à feuille.

13. Système d'administration selon l'une des revendications précédentes, **caractérisé en ce que** la batterie est prévue dans une poche de la plaque support.

14. Système d'administration selon l'une des revendications précédentes, dans lequel la ou les électrode(s) et/ou la ou les contre-électrode(s) sont formées comme une maille.

15. Système d'administration selon l'une des revendications précédentes, dans lequel la ou les électrode(s) et/ou la ou les contre-électrode(s) sont composées d'un matériau métallique, graphitique ou polymère conducteur électrique.

16. Système d'administration selon l'une des revendications précédentes, sachant que le système est conçu pour une conduction de courant comprise entre 0,01 et 10 mA/cm².

17. Système d'administration selon l'une des revendications précédentes, sachant que le réservoir est formé comme un élément interchangeable.

18. Système d'administration selon au moins une des revendications précédentes, sachant que la plaque support avec une ou des électrode (s) et/ou la membrane avec une ou des contre-électrode(s) sont formées comme un élément interchangeable et/ou des éléments interchangeables.

19. Système d'administration selon au moins une des revendications 1 à 16, sachant que la plaque support avec une ou des électrode(s) et/ou la contre-électrode et/ou l'ensemble de contre-électrodes sont formés comme un élément interchangeable et/ou comme des éléments échangeables.

20. Système d'administration selon au moins une des revendications précédentes, sachant que le contrôleur intégré est formé comme un élément interchangeable.
